(19) Europäisches Patentamt
European Patent Office
Office européen des brevets

(11) **EP 4 767 929 A1**

(12) **EUROPEAN PATENT APPLICATION**

(43) Date of publication:
**01.07.2026 Bulletin 2026/27**

(21) Application number: **25197246.9**

(22) Date of filing: **21.08.2025**

(51) International Patent Classification (IPC):
**A61B 5/024** (2006.01)  **A61B 5/0531** (2021.01)
**A61B 5/16** (2006.01)

(52) Cooperative Patent Classification (CPC):
**A61B 5/02416; A61B 5/02405; A61B 5/0531;
A61B 5/165; A61B 5/7264**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **26.12.2024  CN 202411943706**

(71) Applicant: **Kingfar International Inc.
Beijing 100085 (CN)**

(72) Inventors:
• **ZHAO, Qichao**
  **Beijing, 100085 (CN)**
• **WANG, Qingju**
  **Beijing, 100085 (CN)**

(74) Representative: **Meyer-Dulheuer MD Legal
Patentanwälte PartG mbB
Hanauer Landstr. 287-289
60314 Frankfurt am Main (DE)**

(54) **METHOD AND APPARATUS FOR DETECTION PERSONNEL'S STRESS STATE BASED ON HUMAN FACTORS INTELLIGENCE, AND MEDIUM**

(57)    Provided are a method and an apparatus for detecting personnel's stress state based on human factors intelligence, an electronic device and medium. The method includes: obtaining a PPG signal of a test subject, where the PPG signal of the test subject includes first resting-state data and to-be-tested-state data; performing data slicing on the first resting-state data and the to-be-tested-state data respectively to obtain corresponding first segment data and second segment data, where a length of the first segment data is greater than a length of the second segment data; inputting the first segment data and the second segment data into a trained personnel stress state detection model for prediction to obtain a first EDA prediction result; and determining a stress state of the test subject based on the first EDA prediction result.

```
                                                            ┌─ S11
┌──────────────────────────────────────────┐
│  A PPG signal of a test subject is obtained, and the   │
│  PPG signal of the test subject includes first resting- │
│  state data and to-be-detected-state data               │
└──────────────────────────────────────────┘
                        │
                        ▼
                                                            ┌─ S12
┌──────────────────────────────────────────┐
│  Data slicing is performed on the first resting-state data │
│  and the to-be-detected-state data respectively to obtain  │
│  corresponding first segment data and second segment       │
│  data, and a length of the first segment data is greater   │
│  than a length of the second segment data                  │
└──────────────────────────────────────────┘
                        │
                        ▼
                                                            ┌─ S13
┌──────────────────────────────────────────┐
│  The first segment data and the second segment data is     │
│  input into a trained personnel stress state detection model│
│  for prediction to obtain a first EDA prediction result     │
└──────────────────────────────────────────┘
                        │
                        ▼
                                                            ┌─ S14
┌──────────────────────────────────────────┐
│  A stress state of the test subject is determined based on  │
│  the first EDA prediction result                            │
└──────────────────────────────────────────┘
```

FIG. 1

EP 4 767 929 A1

## Description

## TECHNICAL FIELD

The present disclosure relates to the field of the stress state detection, and in particular, to a method and an apparatus for detecting personnel's stress state based on human factors intelligence, an electronic device, and a medium.

## BACKGROUND

In the related art, electroencephalogram (EEG) data or photoplethysmographic (PPG) data are used to detect personnel's stress state. However, the acquisition of the EEG data needs wearing an EEG acquisition device, the wearing process is relatively cumbersome, and the acquired data is prone to motion interference. Meanwhile, when performing personnel stress state prediction by using PPG data, it is usually necessary to use longer PPG data to extract Heart Rate Variability (HRV) features. The PPG data corresponding to a short-time stress state may be submerged in long-time PPG data, affecting detection precision.

## SUMMARY

The present disclosure provides a method and an apparatus for detecting personnel's stress state based on human factors intelligence, an electronic device, and a medium, which helps to solve the problem of low detection precision for the short-time stress state when detecting the personnel's stress state by using the PPG data.

In a first aspect, the present disclosure provides a method for detecting personnel's stress state based on human factors intelligence, including: obtaining a photoplethysmographic (PPG) signal of a test subject, where the PPG signal of the test subject includes first resting-state data and to-be-tested-state data; performing data slicing on the first resting-state data and the to-be-tested-state data respectively to obtain corresponding first segment data and second segment data, where a length of the first segment data is greater than a length of the second segment data; inputting the first segment data and the second segment data into a trained personnel stress state detection model for prediction to obtain a first electrodermal activity (EDA) prediction result; and determining a stress state of the test subject based on the first EDA prediction result.

In a possible implementation, the inputting the first segment data and the second segment data into a trained personnel stress state detection model for prediction to obtain a first EDA prediction result includes: encoding based on the first segment data to obtain a first encoding result; encoding based on the second segment data to obtain a second encoding result; and decoding based on the first segment data and the second segment data to obtain the first EDA prediction result.

In a possible implementation, the encoding based on the first segment data to obtain the first encoding result includes: performing feature extraction on the first segment data by using a first convolutional neural network to obtain first feature information; and performing self-attention calculation based on the first feature information to obtain the first encoding result.

In a possible implementation, the encoding based on the second segment data to obtain the second encoding result includes: encoding the second segment data by using a second convolutional neural network to obtain the second encoding result.

In a possible implementation, the decoding based on the first encoding result and the second encoding result to obtain the first EDA prediction result includes: performing self-attention calculation based on the second encoding result; performing cross-attention calculation based on a result of the self-attention calculation and the first encoding result to obtain state change information; and converting the state change information into the first EDA prediction result by using a third convolutional neural network.

In a possible implementation, a training process of the personnel stress state detection model includes: obtaining PPG signals and EDA signals of training subjects, where the PPG signals of the training subjects include second resting-state data and first experimental data, the EDA signals of the training subjects include third resting-state data and second experimental data, and the PPG signals and the EDA signals of the training subjects are acquired by a physiological signal acquisition device when the training subjects perform a same stress task; performing data normalization based on the third resting-state data and the second experimental data to obtain normalized EDA data; performing data slicing on the second resting-state data, the first experimental data, and the normalized EDA data respectively to obtain corresponding third segment data, fourth segment data, and fifth segment data, where a length of the third segment data is equal to the length of the first segment data, and lengths of the fourth segment data and the fifth segment data are both equal to the length of the second segment data; encoding based on the third segment data to obtain a third encoding result; encoding based on the fourth segment data to obtain a fourth encoding result; decoding based on the third encoding result and the fourth encoding result to obtain a second EDA prediction result; and calculating a loss value based on the second EDA prediction result and the fifth segment data to adjust a model parameter by using the loss value.

In a possible implementation, the stress state of the test subject includes a low stress state, a medium stress state, and a high stress state, and the determining the stress state of the test subject based on the first EDA prediction result includes: when a value of the first EDA prediction result is within a first preset range, determining that the stress state of the test subject is the low stress state; or when a value of the first EDA prediction result is within a second preset range, the stress state of the test subject is the medium stress state; or when a value of the first EDA prediction result is within a third preset range, determining that the stress state of the test subject is the high stress state.

In a possible implementation, after obtaining the PPG signal of the test subject, the method further includes: filtering the PPG signal.

In a second aspect, the present disclosure provides an apparatus for detecting personnel's stress state based on human factors intelligence, including an acquisition module, a data slicing module, a prediction module, and a state determining module. The acquisition module is configured to obtain a photoplethysmographic (PPG) signal of a test subject. The PPG signal of the test subject includes first resting-state data and to-be-tested-state data. The data slicing module is configured to perform data slicing on the first resting-state data and the to-be-tested-state data respectively to obtain corresponding first segment data and second segment data. A length of the first segment data is greater than a length of the second segment data. The prediction module is configured to input the first segment data and the second segment data into a trained personnel stress state detection model for prediction to obtain a first electrodermal activity (EDA) prediction result. The state determining module is configured to determine a stress state of the test subject based on the first EDA prediction result.

In a third aspect, the present disclosure provides an electronic device, including: a processor and a memory configured to store a computer program. The processor, when running the computer program, is configured to implement the method for detecting personnel's stress state based on human factors intelligence according to the first aspect.

In a fourth aspect, the present disclosure provides a computer-readable storage medium on which a computer program is stored. The computer program, when running on a computer, is configured to implement the method for detecting personnel's stress state based on human factors intelligence according to the first aspect.

Compared with the related art, the present disclosure has at least the following technical effects: according to the method for detecting personnel's stress state based on human factors intelligence provided in the embodiments of the present disclosure, by using the easily collectible PPG signal to detect the personnel's stress state, and by converting the PPG signal into the EDA signal, the PPG signal required for the personnel stress state detection is shorter, which is conducive to improving the detection precision of the short-time stress state. In addition, after converting the PPG signal into the EDA signal, the personnel's stress state can be determined according to the change condition of the EDA signal. The determination process is irrelevant to specific tasks, making it possible to achieve the decoupling of personnel stress state detection and specific tasks. Further, the personnel's baseline data (i.e., resting-state data) is considered in the personnel stress state detection, which can suppress individual differences.

**BRIEF DESCRIPTION OF DRAWINGS**

FIG. 1 is a schematic flowchart of a method for detecting personnel's stress state based on human factors intelligence according to an embodiment of the present disclosure;

FIG. 2 is a schematic flowchart of a prediction process using a trained personnel stress state detection model;

FIG. 3 is a structural schematic diagram of a personnel stress state detection model according to an embodiment of the present disclosure;

FIG. 4 is a schematic flowchart of a training process of a personnel stress state detection model according to an embodiment of the present disclosure;

FIG. 5 is a structural schematic diagram of an apparatus for detecting personnel's stress state based on human factors intelligence according to an embodiment of the present disclosure; and

FIG. 6 is a structural schematic diagram of an electronic device according to an embodiment of the present disclosure.

**DESCRIPTION OF EMBODIMENTS**

In the embodiments of the present disclosure, unless otherwise specified, the character "/" herein generally indicates an "or" relationship between the associated objects. For example, A/B may represent A or B. It should be understood that the term "and/or" describes an association relationship of associated objects, and indicates three relationships, for example, A and/or B can indicate only A, A and B, and only B.

It should be noted that terms such as "first" and "second" in the embodiments of the present disclosure are merely used for distinguishing description, and cannot be understood as indicating or implying relative importance or implicitly indicating the number of indicated technical features, or cannot be understood as indicating or implying an order.

In the embodiments of the present disclosure, "at least one" means one or more, and "a plurality of" means two or more. In addition, "at least one of the following items" and similar expressions thereof refer to any combination of these items, including any combination of single items or plural items. For example, at least one of A, B, or C may represent: A, B, C, a combination of A and B, a combination of A and C, a combination of B and C, or a combination of A, B and C. Each of A, B, and C may be an element, or may be a set including one or more elements.

In the embodiments of the present disclosure, expressions such as "exemplary", "in some embodiments", and "in another embodiment" are used to indicate an example, an illustration, or a description. Any embodiment or design described herein as an "example" should not be construed as preferred or advantageous over other embodiments or designs. To be precise, the use of the term "exemplary" is intended to present concepts in a concrete manner.

In the embodiments of the present disclosure, "of", "relevant", and "corresponding" may sometimes be used interchangeably. It should be noted that when their differences are not emphasized, the meanings they intend to express are consistent. In the embodiments of the present disclosure, communication and transmission may sometimes be used interchangeably. It should be noted that when their differences are not emphasized, the meanings they intend to express are consistent. For example, the transmission may include sending and/or receiving, and may be a noun or a verb.

In the embodiments of the present disclosure, the term "equal to" can be used in conjunction with "greater than", which is applicable to the technical solution adopted when "greater than" is used, and can further be used in conjunction with "less than", which is applicable to the technical solution adopted when "less than" is used. It should be noted that, when "equal to" is used in conjunction with "greater than", it cannot be used in conjunction with "less than"; and when "equal to" is used in conjunction with "less than", it cannot be used in conjunction with "greater than".

In the related art, the personnel stress state detection has the following three problems.

First, current commonly used models for detecting personnel's stress state are often based on data from electroencephalogram (EEG) or photoplethysmographic (PPG) modalities. In practical applications, not all tasks are suitable for wearing the EEG acquisition device during their execution. Even when the EEG acquisition device is wearable, wearing such device will have a certain impact on the subject's state, and the collected data will also be affected by the task (e.g., motion interference). A personnel stress state prediction based on a PPG signal usually needs to extract HRV features. However, extracting HRV features necessitates relatively long-time PPG data, such as 3 minutes or more. As a result, the PPG signal corresponding to a short-time stress state may be submerged in a long-time PPG signal, thereby affecting prediction precision.

Second, existing stress state detection models are often used to detect stress states induced by specific tasks, resulting in insufficient cross-task detection capability.

Third, when the physiological signal is used for determining the stress state of a subject, the individual property of the subject has great influence on the determination result, and the existing personnel stress state detection algorithm is insufficient in consideration of individual differences.

Based on the above problems, an embodiment of the present disclosure provides a method for detecting personnel's stress state based on human factors intelligence.

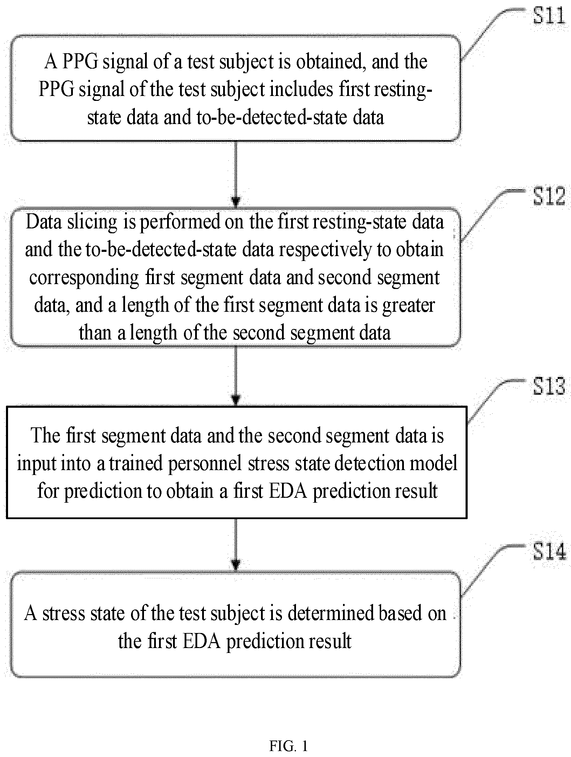

A method for detecting personnel's stress state based on human factors intelligence according to an embodiment of the present disclosure is described with reference to FIG. 1 to FIG. 4.

FIG. 1 is a schematic flowchart of a method for detecting personnel's stress state based on human factors intelligence according to an embodiment of the present disclosure, and the method includes step S11, step S12, step S13, and step S14.

In step S11, a photoplethysmographic (PPG) signal of a test subject is obtained. The PPG signal of the test subject includes first resting-state data and to-be-tested-state data.

The PPG signal of the test subject is collected by using a portable biological information acquisition device or a telemetry device. The PPG signal of the test subject includes the first resting-state data (also referred to as baseline data) and the to-be-tested-state data. The collection process needs to record the start and end time of the first resting-state data, so as to divide the PPG signal of the test subject into the first resting-state data and the to-be-tested-state data according to the start and end time of the first resting-state data.

In an embodiment, after obtaining the PPG signal of the test subject, filtering processing may further be performed on the PPG signal. The effective band range of the PPG signal ranges from 0.5 Hz to 8 Hz, and signals in other frequency bands may be considered as noise signals, which need to be filtered out. Therefore, a 0.5-8 Hz band-pass filtering may be configured for processing to obtain a filtered PPG signal. Filtering the PPG signal can remove noise, thereby improving detection precision.

In step S12, the data slicing is performed on the first resting-state data and the to-be-tested-state data respectively to obtain corresponding first segment data and second segment data. A length of the first segment data is greater than a length of the second segment data.

The first resting-state data and the to-be-tested-state data are segmented. The first resting-state data is segmented into first segment data, and the to-be-tested-state data is segmented into second segment data. A length of the first segment data is greater than a length of the second segment data. For example, first segment data of 60s is extracted from the first resting-state data, and second segment data of 1s is extracted from the to-be-tested-state data. The first resting-state data of a relatively long time is obtained, contributing to the subsequently extraction of the information of the test subject in the resting state, and the to-be-tested data of a relatively short time is obtained, which is conducive to the the personnel stress state detection of the model by using the relatively short-time PPG signal, thereby leading to better real-time prediction of the model. In an embodiment, the length of the first segment data may be 60s, or may be 2min, 3min, or the like. The length of the second segment data may be 1s, 2s, 3s, or the like. The number of the first segment data and the second segment data may be 1 or more, which is not limited in the present disclosure.

For example, the first resting-state data may be segmented into a segment $PPG_{baseseg}$ of 60s, and the to-be-tested-state data may be segmented into 60 segments $PPG_{token}$ each of 1s, then [$PPG_{baseseg}$, $PPG_{token1}$, $PPG_{token2}$, ..., $PPG_{token60}$] is used as an input into the model for prediction.

In step S13, the first segment data and the second segment data are input into a trained personnel stress state detection model for prediction to obtain a first electrodermal activity (EDA) prediction result.

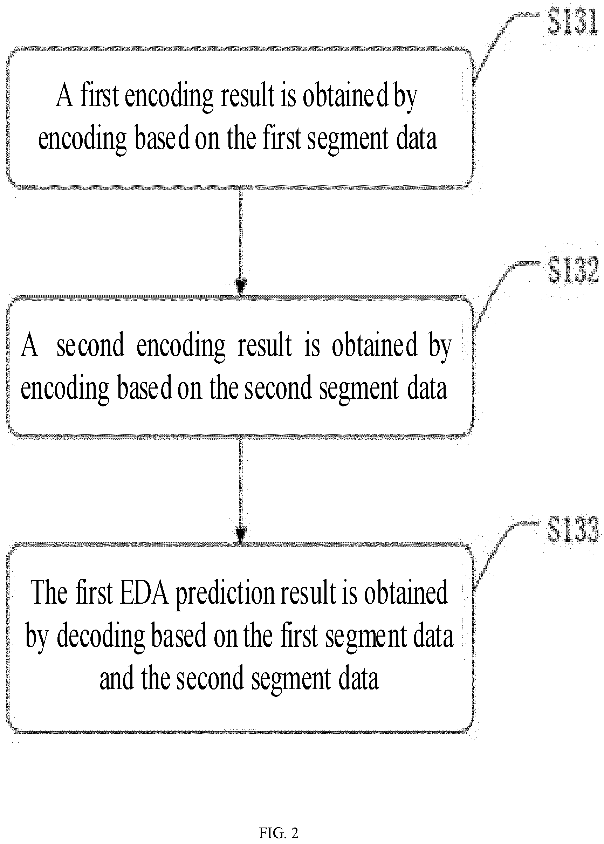

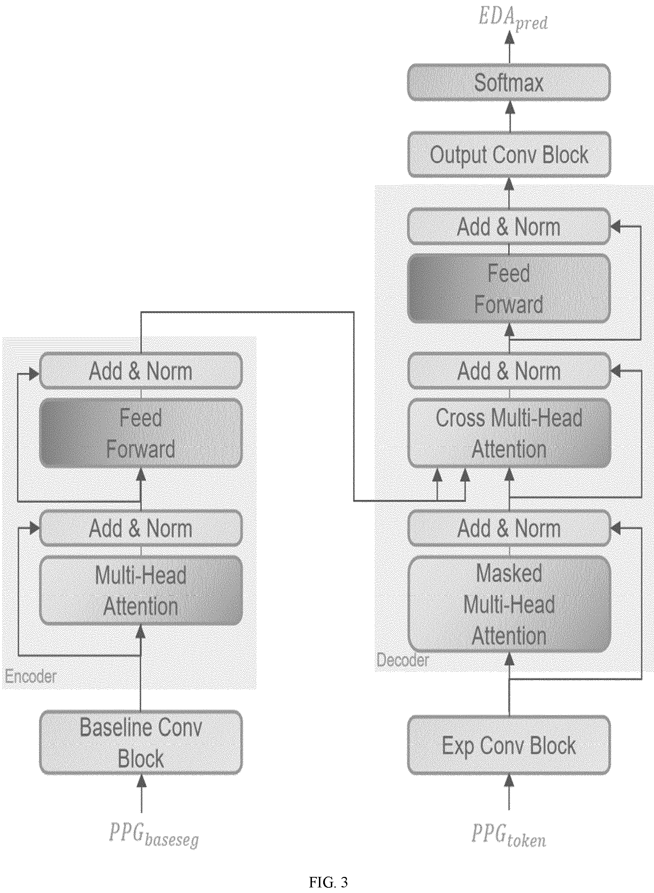

FIG. 2 is a schematic flowchart of a prediction process using a trained personnel stress state detection model. FIG. 3 is a structural schematic diagram of a personnel stress state detection model according to an embodiment of the present disclosure. In an embodiment, the personnel stress state detection model of the present disclosure specifically refers to a transformer model, which includes a first convolutional neural network (Baseline Conv Block shown in FIG. 3), a second convolutional neural network (Exp Conv Block shown in FIG. 3), a third convolutional neural network (Output Conv Block shown in FIG. 3), an encoder (Encoder shown in FIG. 3), and a decoder (Decoder shown in FIG. 3). As shown in FIG.2, the method includes step S131, step S132, and step S133.

In step S131, a first encoding result is obtained by encoding based on the first segment data.

In an embodiment, feature extraction is performed on the first segment data ($PPG_{baseseg}$ shown in FIG. 3) by using the first convolutional neural network to obtain the first feature information. Then, the first feature information is input into the encoder, and self-attention calculation is performed on the multi-head attention module based on the first feature information, so as to obtain the first encoding result. In the calculation, attention will be paid to the information of the whole segment of $PPG_{baseseg}$ to extract the individual information of the test subject and the information of the test subject in the resting state.

In step S132, a second encoding result is obtained by encoding based on the second segment data.

In an embodiment, the second segment data ($PPG_{token}$ shown in FIG. 3) is encoded by using the second convolutional neural network to obtain the second encoding result.

In step S133, the first EDA prediction result is obtained by decoding based on the first segment data and the second segment data.

In an embodiment, the second encoding result is input into a masked multi-head attention module of the decoder, and the self-attention calculation is performed based on the second encoding result. Then, a result of the self-attention calculation and the first encoding result are input into a cross multi-head attention module, and the cross-attention calculation is performed based on the result of the self-attention calculation and the first encoding result, so as to obtain state change information. Finally, the state change information is converted into the first EDA prediction result ($EDA_{pred}$ shown in FIG. 3) by using the third convolutional neural network.

This calculation process takes into account both the baseline-state PPG signal (i.e., $PPG_{baseseg}$) and the PPG signal of the to-be-tested state (i.e., $PPG_{token}$), so that the state change information of the test subject is extracted while suppressing individual differences to a certain extent. Finally, the calculation result is input into the convolutional neural network, so that the state change information with individual differences suppressed is converted into the EDA signal.

The structure of the personnel stress state detection model shown in FIG. 3 further includes: a Feed Forward layer (a feedforward neural network layer), an Add &Norm module (a residual connection and layer normalization operation module), and a softmax layer. The Feed Forward layer is configured to perform nonlinear transformation on a calculation result output by the multi-head attention module. By combining the Feed Forward layer and the multi-head attention module, more comprehensive information can be obtained. Each layer (such as the multi-head attention layer and the feedforward neural network layer) in the model is followed by a residual connection and layer normalization operation module, which is used to add an output of each layer to an input thereof to form a residual connection, and then performs layer normalization. The softmax layer is used to convert an output of the decoder into a final prediction result.

In step S14, a stress state of the test subject is determined based on the first EDA prediction result.

In a possible embodiment, the stress state of the test subject includes a low stress state, a medium stress state, and a high stress state, and the determining the stress state of the test subject based on the first EDA prediction result includes: when a value of the first EDA prediction result is within a first preset range, determining that the stress state of the test subject is the low stress state; or when a value of the first EDA prediction result is within a second preset range, determining that the stress state of the test subject is the medium stress state; or when a value of the first EDA prediction result is within a third preset range, determining that the stress state of the test subject is the high stress state.

For example, if the value of the first EDA prediction result ranges from 0.6 to 1, it is considered that the test subject is in the high stress state. If the value of the first EDA prediction result ranges from 0.3 to 0.6, it is considered that the test subject is in the medium stress state. If the value of the first EDA prediction result ranges from 0 to 0.3, it is considered that the test subject is in the low stress state.

The personnel's stress state is determined according to the model prediction result $EDA_{pred}$. The prediction result $EDA_{pred}$ of the model is the relative change of the personnel's electrodermal activity. By setting different thresholds, different divisions of stress levels can be obtained. Further, the threshold may also be adjusted according to the difficulty of the experiment task. For example, when the task difficulty is relatively low, a relatively low threshold may be set to improve sensitivity. When the task difficulty is relatively high, the threshold may be increased to improve specificity.

According to the present disclosure, by using the easily collectible PPG signal to detect the personnel's stress state, and by converting the PPG signal into the EDA signal, the PPG signal required for personnel stress state detection is shorter, which is conducive to improving the detection precision of the short-time stress state. In addition, after converting the PPG signal into the EDA signal, the personnel's stress state can be determined according to the change condition of the EDA signal. The determination process is irrelevant to specific tasks, and the model can cross different tasks or situations to detect the personnel's stress state, making it possible to achieve the decoupling of personnel stress state detection and specific tasks. This is because the EDA signal directly reflects the physiological stress response of the human body, rather than relying on the induction of specific tasks. Furthermore, the personnel baseline data (i.e., resting-state data) is considered in the personnel stress state detection, and the analysis result is the relative change of the PPG signal. Therefore, individual differences can be suppressed by using the baseline data.

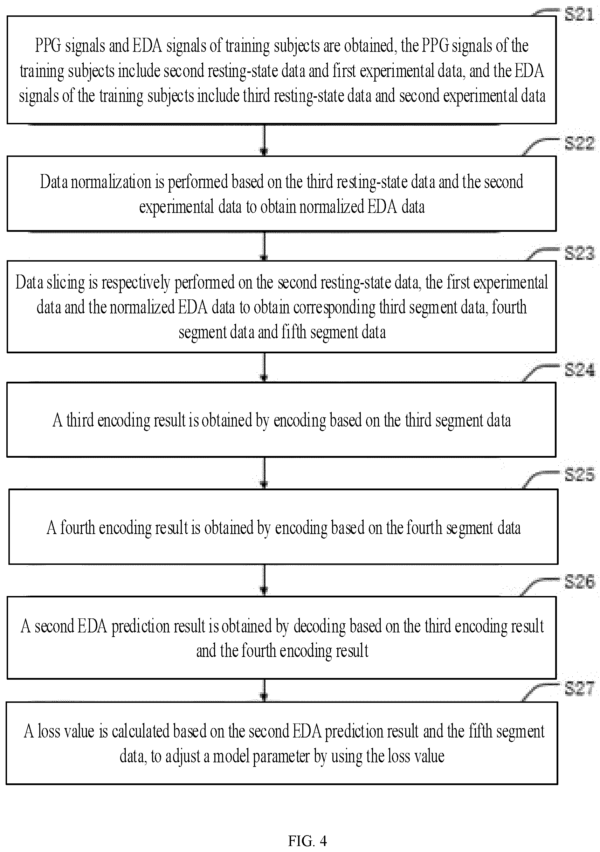

In the present disclosure, a training process of a personnel stress state detection model is shown in FIG. 4, which is a schematic flowchart of a training process of a personnel stress state detection model according to an embodiment of the present disclosure, and the training process specifically includes step S21, step S22, step S23, step S24, step S25, step S26, and step S27:

In step S21, PPG signals and EDA signals of training subjects are obtained. The PPG signal of the training subject includes second resting-state data and first experimental data, and the EDA signal of the training subject includes third resting-state data and second experimental data.

A portable biological information acquisition device (also referred to as a physiological signal acquisition device) or a telemetry device is used to obtain the PPG signal of the training subject, and the portable biological information acquisition device is configured to obtain the EDA signal of the training subject. The PPG signals and the EDA signals of the training subjects are synchronously acquired by a physiological signal acquisition device when the training subjects perform a same stress task. That is, when the training subjects perform the same stress task, the start and end time of the resting-state of the EDA signal is the same as those of the PPG signal, and the start and end time of the experimental data acquisition of the EDA signal is the same as those of the PPG signal.

The collection process needs to record start and end time of the second resting-state data and start and end time of the third resting-state data, so as to divide the PPG signal of the training subject into the second resting-state data and the first experimental data according to the start and end time of the second resting-state data, and divide the EDA signal of the training subject into the third resting-state data and the second experimental data according to the start and end time of the third resting-state data.

In an embodiment, the collected PPG signal and EDA signal are filtered. The PPG signal may be processed by using the 0.5-8 Hz band-pass filtering to obtain the filtered PPG signal. An effective frequency of the EDA signal is relatively low, so the EDA signal is processed by using a 3Hz low-pass filtering to obtain the filtered EDA signal.

In step S22, the data normalization is performed based on the third resting-state data and the second experimental data to obtain normalized EDA data.

Since the EDA data of the training subjects vary, it is necessary to normalize the EDA data to effectively suppress individual differences. The specific expression relationship is as follows:

$$EDA_{norm} = \frac{EDA_{exp}}{mean(EDA_{base}) * 10}.$$

$EDA_{norm}$ refers to the normalized EDA data. $EDA_{exp}$ refers to the second experimental data. $EDA_{base}$ refers to the third resting-state data. Dividing the second experimental data by the mean of the baseline data (i.e., the third resting-state data) and then dividing by 10 is to normalize the data to the range from 0 to 1.

By normalizing the EDA data, differences in baseline levels of different individuals can be eliminated, and the data is adjusted to a common scale, so as to compare data of different individuals or analyze data changes of the same individual under different conditions, so that the data is more comparable. The data is scaled to the range from 0 to 1, which helps the subsequent model training. Additionally, the normalized data can improve the convergence speed and performance of the model.

In step S23, data slicing is respectively performed on the second resting-state data, the first experimental data and the normalized EDA data to obtain corresponding third segment data, fourth segment data and fifth segment data.

After the second resting-state data, the first experimental data, and the normalized EDA data are segmented, the second resting-state data is segmented into third segment data, the first experimental data is segmented into fourth segment data, and the normalized EDA data is segmented into fifth segment data. A length of the third segment data is equal to the length of the first segment data, and lengths of the fourth segment data and the fifth segment data are both equal to the length of the second segment data, which meets the requirement of the model on a size of the input data. For example, the length of the third segment data is equal to the length of the first segment data, both of which are 60s. Lengths of the fourth segment data and the fifth segment data are equal to the length of the second segment data, both of which are 1s. The number of the third segment data, the fourth segment data, and the fifth segment data may be 1 or more, which is not limited in the present disclosure.

In an optional implementation, the second resting-state data is segmented into a third segment data $PPG_{sbaseseg}$ of 60s. The first experimental data is segmented into 60 fourth segment data $PPG_{stoken}$ each of 1s. The normalized EDA data is segmented into 60 fifth segment data $EDA_{token}$ each of 1s. Then $[PPG_{sbaseseg}, PPG_{stoken1}, PPG_{stoken2}, ..., PPG_{stoken60}, EDA_{token1}, EDA_{token2}, ..., EDA_{token60}]$ is used as input data for model training.

In step S24, a third encoding result is obtained by encoding based on the third segment data.

A specific implementation principle refers to the foregoing step S131, and details are not described herein again.

In step S25, a fourth encoding result is obtained by encoding based on the fourth segment data.

A specific implementation principle refers to the foregoing step S132, and details are not described herein again.

In step S26, the second EDA prediction result is obtained by decoding based on the third encoding result and the fourth encoding result.

A specific implementation principle refers to the foregoing step S133, and details are not described herein again.

In step S27, a loss value is calculated based on the second EDA prediction result and the fifth segment data to adjust a model parameter by using the loss value.

In an embodiment, the second EDA prediction result is obtained through calculation in step S26, the second EDA prediction result is compared with the fifth segment data $EDA_{token}$, a loss value is calculated and back propagated, so that model parameters are adjusted by using the loss value.

In an optional embodiment, for a set of data $[PPG_{sbaseseg}, PPG_{stoken1}, PPG_{stoken2}, ..., PPG_{stoken60}, EDA_{token1}, EDA_{token2}, ..., EDA_{token60}]$, step 24 is first performed to encode $PPG_{sbaseseg}$ to obtain a third encoding result, then step 25 is performed to encode $PPG_{gtoken1}$ to obtain a fourth encoding result, then step 26 is performed to decode the third encoding result and the fourth encoding result to obtain a second EDA prediction result, and finally, the second EDA prediction result is compared with $EDA_{token1}$ to calculate the loss value to complete a first training. After the first training is completed, the input of step S25 is updated as $[PPG_{stoken1}, PPG_{stoken2}]$, steps S24 to S26 are repeated, the prediction result obtained in step S26 is compared with $EDA_{token2}$ to calculate the loss value, and the second training is completed. The above process is repeated until 60 $PPG_{stoken}$s are trained.

According to the present disclosure, the PPG data is encoded based on the Transformer model and then decoded into the EDA data. Personnel stress state detection is carried out by utilizing the EDA data. Baseline data is effectively utilized in the decoding and encoding process of the model, which suppresses individual differences in personnel physiological signals to a certain extent. Moreover, the PPG signal is converted into the EDA signal with stronger correlation with the stress state, and the personnel's stress state is determined according to the change condition of the EDA signal. After converting the PPG signal into the EDA signal, the determination process is irrelevant to the specific tasks, making it possible to achieve the decoupling of personnel stress state detection and specific tasks. In addition, the PPG signal required for personnel state detection is shorter, which is conducive to identification of the short-time stress state.

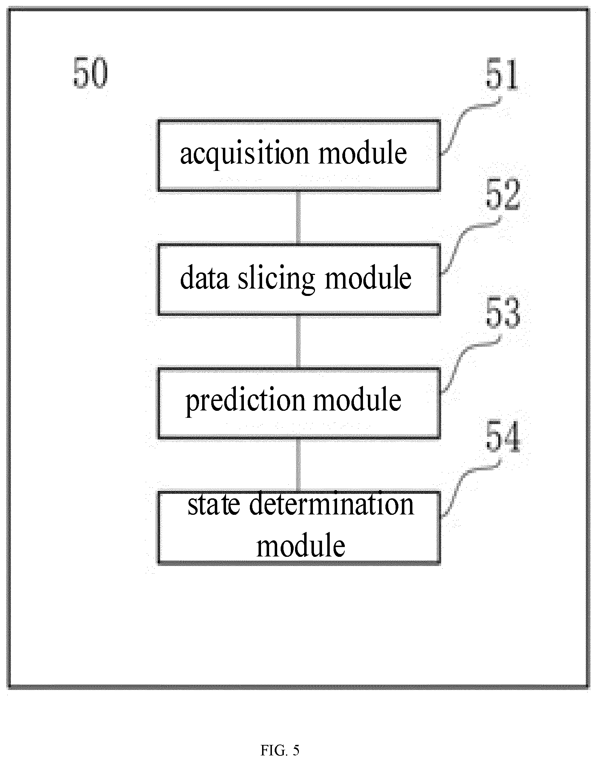

Based on the same idea, FIG. 5 is a structural schematic diagram of an apparatus for detecting personnel's stress state based on human factors intelligence according to an embodiment of the present disclosure. As shown in FIG. 5, the apparatus 50 for detecting personnel's stress state based on human factors intelligence may include: an acquisition module 51, a data slicing module 52, a prediction module 53, and a state determination module 54.

The acquisition module 51 is configured to obtain a PPG signal of a test subject. The PPG signal of the test subject includes first resting-state data and to-be-tested-state data.

The data slicing module 52 is configured to perform data slicing on the first resting-state data and the to-be-tested-state data respectively to obtain corresponding first segment data and second segment data. A length of the first segment data is greater than a length of the second segment data.

The prediction module 53 is configured to input the first segment data and the second segment data into a trained personnel stress state detection model for prediction to obtain a first EDA prediction result.

The state determination module 54 is configured to determine a stress state of the test subject based on the first EDA prediction result.

In a possible implementation, the prediction module 53 may be further configured for: encoding based on the first segment data to obtain a first encoding result; encoding based on the second segment data to obtain a second encoding result; and decoding based on the first segment data and the second segment data to obtain the first EDA prediction result.

In a possible implementation, the encoding based on the first segment data to obtain a first encoding result includes: performing feature extraction on the first segment data by using a first convolutional neural network to obtain first feature information; and performing self-attention calculation based on the first feature information to obtain the first encoding result.

In a possible implementation, the encoding based on the second segment data to obtain a second encoding result includes: encoding the second segment data by using a second convolutional neural network to obtain the second encoding result.

In a possible implementation, the decoding based on the first encoding result and the second encoding result to obtain the first EDA prediction result includes: performing self-attention calculation based on the second encoding result; and performing cross-attention calculation based on a result of the self-attention calculation and the first encoding result, to obtain state change information; and converting the state change information into the first EDA prediction result by using a third convolutional neural network.

In a possible implementation, a training process of the personnel stress state detection model includes: obtaining PPG signals and EDA signals of training subjects, where the PPG signals of the training subjects include second resting-state data and first experimental data, the EDA signals of the training subjects include third resting-state data and second experimental data, and the PPG signals and the EDA signals of the include are synchronously acquired by a physiological signal acquisition device when the training subjects perform a same stress task; performing data normalization based on the third resting-state data and the second experimental data to obtain normalized EDA data; performing data slicing on the second resting-state data, the first experimental data, and the normalized EDA data respectively to obtain corresponding third segment data, fourth segment data, and fifth segment data, where a length of the third segment data is equal to the length of the first segment data, and lengths of the fourth segment data and the fifth segment data are both equal to the length of the second segment data; encoding based on the third segment data to obtain a third encoding result; encoding based on the fourth segment data to obtain a fourth encoding result; decoding based on the third encoding result and the fourth encoding result to obtain a second EDA prediction result; and calculating a loss value based on the second EDA prediction result and the fifth segment data, to adjust a model parameter by using the loss value.

In a possible implementation, the stress state of the test subject includes a low stress state, a medium stress state, and a high stress state. The state determination module 54 may be further configured for determining that: the stress state of the test subject is the low stress state when a value of the first EDA prediction result is within a first preset range; or the stress state of the test subject is the medium stress state when a value of the first EDA prediction result is within a second preset range; or the stress state of the test subject is the high stress state when a value of the first EDA prediction result is within a third preset range.

In a possible implementation, the apparatus further includes a filtering module. The filtering module is configured to filter processing on the PPG signal after obtaining the PPG signal of the test subject.

The apparatus 50 for detecting personnel's stress state based on human factors intelligence provided in the embodiment shown in FIG. 5 may be configured to execute the technical solutions of the method embodiments shown in the present disclosure, and for the implementation principle and the technical effect thereof, reference may be further made to related descriptions in the method embodiments.

It should be understood that the division of the modules of the apparatus 50 for detecting personnel's stress state based on human factors intelligence shown in FIG. 5 is merely a logical function division, and may be completely or partially integrated into one physical entity in actual implementation, or may be physically separated. In addition, all the modules may be implemented in a form of software called by a processing element; or all the modules may be implemented in a form of hardware; or some modules may be implemented in a form of software called by the processing element, and some modules are implemented in a form of hardware. For example, the acquisition module may be an independent processing element, or may be integrated into a chip of the electronic device. The other modules are implemented similarly. In addition, all or part of these modules may be integrated together, or may be separately implemented. In an implementation process, each step in the foregoing method or each module may be completed by an integrated logic circuit of hardware in a processor element or an instruction in a form of software.

For example, the above modules may be one or more integrated circuits configured to implement the above methods, such as: one or more application specific integrated circuits (hereinafter referred to as ASIC), or one or more digital signal processors (hereinafter referred to as DSP), or one or more field programmable gate arrays (hereinafter referred to as FPGA), or the like. For another example, these modules may be integrated together and implemented in a form of a system-on-a-chip (hereinafter referred to as SOC).

In the above embodiments, the involved processor may include, for example, a central processing unit (CPU), a microcontroller, or a digital signal processor (DSP), and may also include a graphics processing unit (GPU), an embedded neural-network process units (hereinafter referred to as NPU), and an image signal processing (hereinafter referred to as ISP). The processor may further include necessary hardware accelerators or logic processing hardware circuits, such as ASIC, or one or more integrated circuits for controlling the execution of the program of the technical solutions of the present disclosure. Further, the processor may have the function of operating one or more software programs that may be stored in the storage medium.

An embodiment of the present disclosure further provides a computer-readable storage medium on which a computer program is stored. The computer program, when running on a computer, is configured to implement the method provided in the embodiments of the present disclosure.

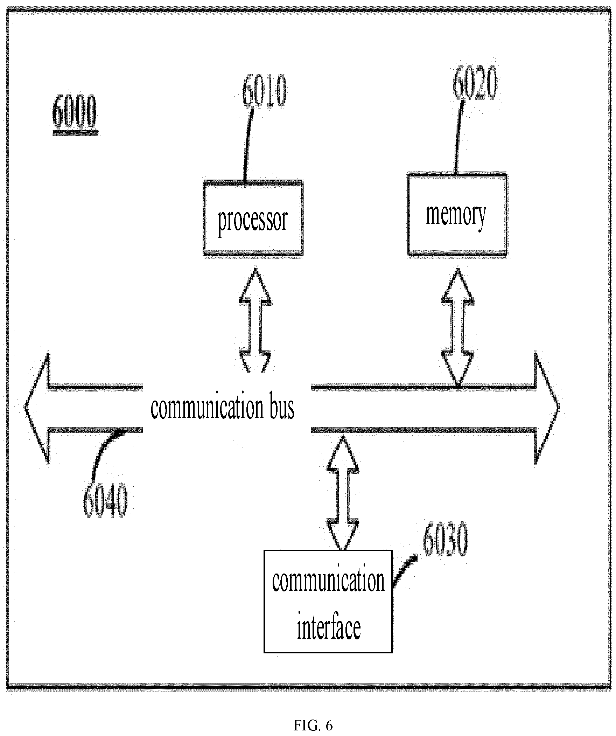

An exemplary electronic device provided in the embodiments of the present disclosure is further described with reference to FIG. 6, which is a schematic structural diagram of an electronic device 6000.

The electronic device 6000 may include: at least one processor; and at least one memory communicatively connected to the processor. The memory stores program instructions executed by the processor, and the processor can execute the method for detecting personnel's stress state based on human factors intelligence provided by the embodiments of the present disclosure by calling the above program instructions.

FIG. 6 shows a block diagram of an exemplary electronic device 6000 suitable for implementing embodiments of the present disclosure. The electronic device 6000 shown in FIG. 6 is merely an example, and has no limitation to the function and scope of use of the embodiments of the present disclosure.

As shown in FIG. 6, the electronic device 6000 is represented in a form of a general-purpose computing device. Components of the electronic device 6000 may include, but are not limited to: one or more processors 6010, a memory 6020, a communication bus 6040 connecting different system components (including the memory 6020 and the processor 6010), and a communication interface 6030.

The communication bus 6040 represents one or more of several types of bus structures, including a memory bus or a memory controller, a peripheral bus, a graphics acceleration port, a processor, or a local bus using any of a variety of bus structures. For example, these architectures include but are not limited to industry standard architecture (hereinafter referred to as ISA) bus, micro channel architecture (hereinafter referred to as MAC) bus, enhanced ISA bus, video electronics standards association (hereinafter referred to as VESA) local bus, and peripheral component interconnect (hereinafter referred to as PCI) Bus.

The electronic device 6000 typically includes a variety of computer system readable media. These media may be any available media that can be accessed by the electronic device, including volatile and non-volatile media, removable and non-removable media.

The memory 6020 may include a computer system readable medium in a form of a volatile memory, such as a random-access memory (hereinafter referred to as RAM) and/or a cache memory. The electronic device may further include other removable/non-removable, and volatile/non-volatile computer system storage media. Although not shown in FIG. 6, a disk driver for reading and writing a removable non-volatile disk (such as a "floppy disk"), and an optical disk driver for reading and writing a removable non-volatile optical disk (such as compact disc read only memory (hereinafter referred to as CD-ROM), digital video disc read only memory (hereinafter referred to as DVD ROM), or other optical media) can be provided. In these cases, each driver may be connected to communication bus 6040 via one or more data media interfaces. The memory 6020 may include at least one program product having a set (e.g., at least one) of program modules configured to perform functions of the embodiments of the present disclosure.

A program/utility tool with a set (at least one) of program modules may be stored in the memory 6020, such program modules includes, but not limited to, an operating system, one or more application programs, other program modules and program data, each or some combination of which may include an implementation of a network environment. The program modules generally perform functions and/or methods in the embodiments described in the present disclosure.

The electronic device 6000 may also communicate with one or more external devices (such as a keyboard, a pointing device, a display, etc.), one or more devices that enable a user to interact with the electronic device, and/or any device (such as a network card, a modem, etc.) that enables the electronic device to communicate with one or more other computing devices. Such communication may be performed through the communication interface 6030. Further, the electronic device 6000 may further communicate with one or more networks (such as a local area network (hereinafter referred to as LAN), a wide area network (hereinafter referred to as WAN) and/or a public network (such as the Internet)) through a network adapter (not shown in FIG. 6), which can communicate with other modules of the electronic device through the communication bus 6040. It should be understood that although not shown in FIG. 6, other hardware and/or software modules can be used in conjunction with the electronic device 6000, including but not limited to: a microcode, a device driver, a redundant processing unit, an external disk drive array, and a redundant array of independent drives (hereinafter referred to as RAID) system, a tape driver, and a data backup storage system.

By running a program stored in the memory 6020, the processor 6010 executes various functional applications and data processing, such as implement the method provided in the embodiments of the present disclosure.

It is understandable that the interface connection relationships between the various modules illustrated in the embodiments of the present disclosure are merely schematic illustrations and do not constitute a structural limitation on the electronic device 6000. In other embodiments of the present disclosure, the electronic device 6000 may also adopt different interface connection modes from those in the above embodiments, or a combination of multiple interface connection modes.

In the above embodiments, the involved processor may include, for example, a central processing unit (CPU), a microcontroller, or a digital signal processor (DSP), and may also include a graphics processing unit (GPU), an embedded neural-network process units (hereinafter referred to as NPU), and an image signal processing (hereinafter referred to as ISP). The processor may further include necessary hardware accelerators or logic processing hardware circuits, such as ASIC, or one or more integrated circuits for controlling the execution of the program of the technical solutions of the present disclosure. Further, the processor may have the function of operating one or more software programs that may be stored in the storage medium.

Those skilled in the art may recognize that the units and algorithm steps described in the embodiments disclosed herein can be implemented using electronic hardware, and a combination of electronic hardware and computer software. Whether these functions are executed in hardware or software depends on the specific application and design constraints of the technical solutions. Those skilled in the art may use different methods to implement the described functions for each specific application, but such implementation should not be considered to be beyond a scope of the present disclosure.

Those skilled in the art should clearly understand that, for convenience and conciseness of description, the specific operating processes of the above-described system, apparatus and unit can refer to the corresponding process in the above method embodiments, which will not be repeated herein.

In some embodiments provided in the present disclosure, if any function is implemented in a form of a software function unit and is sold or used as an independent product, the function may be stored in a computer-readable storage medium. Based on such understanding, the technical solution of the present disclosure can essentially, or the part that contributes to the related art, or a part of the technical solution be embodied in the form of a software product. The computer software product is stored in the storage medium and includes a number of instructions for enabling a computer device, which may be a personal computer, a server, or a network device, etc., to execute all or some of the steps of the method described in each embodiment of the present disclosure. The above storage medium includes: various media that may store program codes, such as universal serial bus (USB) flash drive, mobile hard disk, read-only memory (hereinafter referred to as ROM), random access memory (hereinafter referred to as RAM), magnetic disk, or optical disk.

The above descriptions are merely specific implementations of the present disclosure. Any person skilled in the art can easily think of changes or substitutions within the technical scope disclosed in the present disclosure, and they should be covered within the protection scope of the present disclosure. The protection scope of the present disclosure should be based on the protection scope of the claims.

**Claims**

1. A method for detecting personnel's stress state based on human factors intelligence, comprising:

   obtaining a photoplethysmographic, PPG, signal of a test subject, wherein the PPG signal of the test subject comprises first resting-state data and to-be-tested-state data;
   performing data slicing on the first resting-state data and the to-be-tested-state data respectively to obtain corresponding first segment data and second segment data, wherein a length of the first segment data is greater than a length of the second segment data;
   inputting the first segment data and the second segment data into a trained personnel stress state detection model for prediction to obtain a first electrodermal activity, EDA, prediction result; and
   determining a stress state of the test subject based on the first EDA prediction result.

2. The method according to claim 1, wherein the inputting the first segment data and the second segment data into the trained personnel stress state detection model for prediction to obtain the first EDA prediction result, comprises:

   encoding based on the first segment data to obtain a first encoding result;
   encoding based on the second segment data to obtain a second encoding result; and
   decoding based on the first segment data and the second segment data to obtain the first EDA prediction result.

3. The method according to claim 2, wherein the encoding based on the first segment data to obtain the first encoding result, comprises:

   performing feature extraction on the first segment data by using a first convolutional neural network to obtain first feature information; and
   performing self-attention calculation based on the first feature information to obtain the first encoding result.

4. The method according to claim 3, wherein the encoding based on the second segment data to obtain the second encoding result, comprises:
   encoding the second segment data by using a second convolutional neural network to obtain the second encoding result.

5. The method according to claim 4, wherein the decoding based on the first segment data and the second segment data to obtain the first EDA prediction result, comprises:

performing self-attention calculation based on the second encoding result;
performing cross-attention calculation based on a result of the self-attention calculation and the first encoding result to obtain state change information; and
converting the state change information into the first EDA prediction result by using a third convolutional neural network.

6. The method according to claim 1, wherein a training process of the personnel stress state detection model comprises:

obtaining PPG signals and EDA signals of training subjects, where the PPG signals of the training subjects comprise second resting-state data and first experimental data, the EDA signals of the training subjects comprise third resting-state data and second experimental data, and the PPG signals and the EDA signals of the training subjects are simultaneously acquired by a physiological signal acquisition device when the training subjects perform a same stress task;
performing data normalization based on the third resting-state data and the second experimental data to obtain normalized EDA data;
performing data slicing on the second resting-state data, the first experimental data, and the normalized EDA data respectively to obtain corresponding third segment data, fourth segment data, and fifth segment data, wherein a length of the third segment data is equal to the length of the first segment data, and a length of the fourth segment data and a length of the fifth segment data are both equal to the length of the second segment data;
encoding based on the third segment data to obtain a third encoding result;
encoding based on the fourth segment data to obtain a fourth encoding result;
decoding based on the third encoding result and the fourth encoding result to obtain a second EDA prediction result; and
calculating a loss value based on the second EDA prediction result and the fifth segment data to adjust a model parameter by using the loss value.

7. The method according to claim 1, wherein the stress state of the test subject comprises a low stress state, a medium stress state, or a high stress state, and the determining the stress state of the test subject based on the first EDA prediction result comprises:

determining that the stress state of the subject is the low stress state when a value of the first EDA prediction result is within a first preset range; or
determining that the stress state of the subject is the medium stress state when a value of the first EDA prediction result is within a second preset range; or
determining that the stress state of the subject is the high stress state when a value of the first EDA prediction result is within a third preset range.

8. The method according to any one of claims 1 to 7, wherein after obtaining the PPG signal of the test subject, the method further comprises:
filtering the PPG signal.

9. An apparatus for detecting personnel's stress state based on human factors intelligence, comprising:

an acquisition module configured to obtain a photoplethysmographic, PPG, signal of a subject, wherein the PPG signal of the subject comprises first resting-state data and to-be-tested-state data;
a data slicing module configured to perform data slicing on the first resting-state data and the to-be-tested-state data respectively to obtain corresponding first segment data and second segment data, wherein a length of the first segment data is greater than a length of the second segment data;
a prediction module configured to input the first segment data and the second segment data into a trained personnel stress state detection model for prediction to obtain a first electrodermal activity ,EDA, prediction result; and
a state determination module configured to determine a stress state of the subject based on the first EDA prediction result.

10. The apparatus according to the claim 9, wherein the prediction module is configured to input the first segment data and the second segment data into a trained personnel stress state detection model for prediction to obtain a first electrodermal activity, EDA, prediction result, comprising:

encoding based on the first segment data to obtain a first encoding result;
encoding based on the second segment data to obtain a second encoding result; and
decoding based on the first segment data and the second segment data to obtain the first EDA prediction result.

**11.** The apparatus according to the claim 10, wherein the prediction module is configured to encode based on the first segment data to obtain the first encoding result, comprising:

performing feature extraction on the first segment data by using a first convolutional neural network to obtain first feature information; and performing self-attention calculation based on the first feature information to obtain the first encoding result,
wherein the prediction module is configured to encode based on the second segment data to obtain the second encoding result, comprising: encoding the second segment data by using a second convolutional neural network to obtain the second encoding result.

**12.** The apparatus according to the claim 11, wherein the prediction module is configured to decode based on the first segment data and the second segment data to obtain the first EDA prediction result, comprising:

performing self-attention calculation based on the second encoding result;
performing cross-attention calculation based on a result of the self-attention calculation and the first encoding result to obtain state change information; and
converting the state change information into the first EDA prediction result by using a third convolutional neural network.

**13.** The apparatus according to the claim 9, wherein the apparatus further comprises:
a training module configured to train the personnel stress state detection model comprising:

obtaining PPG signals and EDA signals of training subjects, where the PPG signals of the training subjects comprise second resting-state data and first experimental data, the EDA signals of the training subjects comprise third resting-state data and second experimental data, and the PPG signals and the EDA signals of the training subjects are simultaneously acquired by a physiological signal acquisition device when the training subjects perform a same stress task;
performing data normalization based on the third resting-state data and the second experimental data to obtain normalized EDA data;
performing data slicing on the second resting-state data, the first experimental data, and the normalized EDA data respectively to obtain corresponding third segment data, fourth segment data, and fifth segment data, wherein a length of the third segment data is equal to the length of the first segment data, and a length of the fourth segment data and a length of the fifth segment data are both equal to the length of the second segment data;
encoding based on the third segment data to obtain a third encoding result;
encoding based on the fourth segment data to obtain a fourth encoding result;
decoding based on the third encoding result and the fourth encoding result to obtain a second EDA prediction result; and
calculating a loss value based on the second EDA prediction result and the fifth segment data to adjust a model parameter by using the loss value.

**14.** An electronic device, comprising: a processor and a memory configured to store a computer program, wherein the processor, when running the computer program, is configured to implement the method for detecting personnel's stress state based on human factors intelligence according to any one of claims 1 to 8.

**15.** A computer-readable storage medium on which a computer program is stored, wherein the computer program, when running on a computer, is configured to implement the method for detecting personnel's tress state based on human factors intelligence according to any one of claims 1 to 8.

S11

A PPG signal of a test subject is obtained, and the PPG signal of the test subject includes first resting-state data and to-be-detected-state data

S12

Data slicing is performed on the first resting-state data and the to-be-detected-state data respectively to obtain corresponding first segment data and second segment data, and a length of the first segment data is greater than a length of the second segment data

S13

The first segment data and the second segment data is input into a trained personnel stress state detection model for prediction to obtain a first EDA prediction result

S14

A stress state of the test subject is determined based on the first EDA prediction result

FIG. 1

S131

A first encoding result is obtained by encoding based on the first segment data

S132

A second encoding result is obtained by encoding based on the second segment data

S133

The first EDA prediction result is obtained by decoding based on the first segment data and the second segment data

FIG. 2

$EDA_{pred}$

Softmax

Output Conv Block

Add & Norm

Feed Forward

Add & Norm

Cross Multi-Head Attention

Add & Norm

Masked Multi-Head Attention

Decoder

Exp Conv Block

$PPG_{token}$

Add & Norm

Feed Forward

Add & Norm

Multi-Head Attention

Encoder

Baseline Conv Block

$PPG_{baseseg}$

FIG. 3

PPG signals and EDA signals of training subjects are obtained, the PPG signals of the training subjects include second resting-state data and first experimental data, and the EDA signals of the training subjects include third resting-state data and second experimental data

S21

Data normalization is performed based on the third resting-state data and the second experimental data to obtain normalized EDA data

S22

Data slicing is respectively performed on the second resting-state data, the first experimental data and the normalized EDA data to obtain corresponding third segment data, fourth segment data and fifth segment data

S23

A third encoding result is obtained by encoding based on the third segment data

S24

A fourth encoding result is obtained by encoding based on the fourth segment data

S25

A second EDA prediction result is obtained by decoding based on the third encoding result and the fourth encoding result

S26

A loss value is calculated based on the second EDA prediction result and the fifth segment data, to adjust a model parameter by using the loss value

S27

FIG. 4

50

51

acquisition module

52

data slicing module

53

prediction module

54

state determination module

FIG. 5

FIG. 6

Europäisches
Patentamt

European
Patent Office

Office européen
des brevets

## EUROPEAN SEARCH REPORT

**Application Number**

EP 25 19 7246

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BRAUN BJÖRN ET AL: "Video-based sympathetic arousal assessment via peripheral blood flow estimation", BIOMEDICAL OPTICS EXPRESS, vol. 14, no. 12, 30 November 2023 (2023-11-30), page 6607, XP093397615, United States ISSN: 2156-7085, DOI: 10.1364/boe.507949 *Conclusion*; page 6608 - page 6609 * abstract * | 1-15 | INV. A61B5/024 A61B5/0531 A61B5/16 |
| | ----- | | |
| X,P | CN 119 903 299 A (KINGFAR INT INC) 29 April 2025 (2025-04-29) * the whole document * | 1-15 | |
| | ----- | | |

| | |
|---|---|
| | **TECHNICAL FIELDS SEARCHED (IPC)** |
| | A61B |

The present search report has been drawn up for all claims

| Place of search | Date of completion of the search | Examiner |
|---|---|---|
| Berlin | 12 May 2026 | Clevorn, Jens |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 19 7246

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2026

| Patent document cited in search report | Publication date | Patent family member(s) | Publication date |
|---|---|---|---|
| CN 119903299 A | 29-04-2025 | NONE | |